# EUROPEAN PATENT APPLICATION

(11) **EP 4 834 213 A1**
(43) Date of publication of application: **04.11.2026**
(21) Application number: 26175406.3
(22) Date of filing: 29.04.2026
(51) Int. Cl.: A61B 5/03, A61B 5/00

(54) **CAPACITIVE CATHETER SYSTEM WITH CAPACITIVE PRESSURE SENSOR AND RELATED METHODS**

(30) Priority: 30.04.2025 US 202519194456
(71) Applicant: Laborie Medical Technologies Corp., Portsmouth, NH 03801 (US)
(72) Inventor: PETERS, Julian, Newmarket (US); BERNIER, Ryan, Portsmouth (US)
(74) Representative: Reddie & Grose LLP

(57) **Abstract**

A catheter assembly is disclosed. The catheter assembly includes a catheter and a sensor assembly. The catheter includes a catheter shaft and an electronics lumen formed in the catheter shaft. The sensor assembly is inserted into the electronics lumen. The sensor assembly includes a rigid PCB, a flexible PCB, a dielectric region, and a cap. The rigid PCB retains a rigid shape. The flexible PCB includes a contact portion joined to the rigid PCB and an offset portion spaced apart from the rigid PCB. The dielectric region is positioned between the rigid PCB and the offset portion of the flexible PCB. The cap includes an encapsulant adjoining at least a portion of the offset portion. The encapsulant is configured to allow a pressure or force applied thereto to transfer to and displace at least a portion of the offset portion towards the rigid PCB.

## Description

### PRIORITY CLAIM

This application claims the benefit of the filing date of United States Patent Application Serial No. 19/194,456, filed April 30, 2025, for "CAPACITIVE CATHETER SYSTEM WITH CAPACITIVE PRESSURE SENSOR AND RELATED METHODS," the disclosure of which is hereby incorporated herein in its entirety by this reference.

### TECHNICAL FIELD

This disclosure relates generally to pressure sensing catheters, and more specifically to electronic catheter systems including a capacitive sensor and related methods.

### BACKGROUND

Pressure sensing catheter systems for measuring pressure within the human body, such as for measuring pressure within various parts of the gastrointestinal tract, include fluid-based catheter systems (e.g., air-based catheter systems and water-based catheter systems) and electronics-based catheter systems. Fluid-based catheter systems rely on a fluid medium to transmit pressure applied to the catheter within the body to a transducer located outside of the body. Fluid-based catheter systems require external setups to ensure external connections for transferring the pressure from the fluid to the transducer are properly formed, the catheter is properly sealed and that air bubbles are not present within the catheter. Fluid-based catheter systems may have a signal delay (as the pressure is transferred along the fluid medium within the catheter to the transducer) and may have accuracy issues (e.g., pressure damping caused by the fluid medium).

When compared to fluid-based catheter systems, electronics-based catheter systems provide multiple performance benefits, such as improved accuracy and frequency response along with an improved ease of use since there is minimal setup involved in fluid-less systems.

However, current electronics-based catheter systems utilize cost prohibitive Micro-Electro-Mechanical System (MEMS) piezoresistive sensor dies. The cost of the MEMS piezoresistive sensor die along with the intricate design and complexity of supporting electronics required for MEMS-based catheter systems makes MEMS-based catheter systems expensive to manufacture. Further, MEMS-based catheter systems often utilize brittle materials that may fail under certain conditions.

### BRIEF SUMMARY

In various embodiments, the present disclosure provides a catheter assembly. The catheter assembly includes a catheter and a sensor assembly. The catheter includes a catheter shaft and an electronics lumen formed in the catheter shaft. The sensor assembly is inserted into the electronics lumen. The sensor assembly includes a rigid PCB, a flexible PCB, a dielectric region, and a cap. The rigid PCB retains a rigid shape. The flexible PCB includes a contact portion joined to the rigid PCB and an offset portion spaced apart from the rigid PCB. The dielectric region is positioned between the rigid PCB and the offset portion of the flexible PCB. The cap includes an encapsulant adjoining at least a portion of the offset portion. The encapsulant is configured to allow a pressure or force applied thereto to transfer to and displace at least a portion of the offset portion towards the rigid PCB.

In various embodiments, the present disclosure provides a catheter system. The catheter system includes a catheter, a sensor assembly, and a measurement device. The catheter includes a catheter shaft and an electronics lumen formed in the catheter shaft. The sensor assembly is inserted into the electronics lumen. The sensor assembly includes a capacitive sensor and an encapsulant. The capacitive sensor includes a rigid printed circuit board (PCB), a flexible PCB, and a dielectric region. The rigid printed PCB retains a rigid shape. The flexible PCB includes a contact portion joined to the rigid PCB, an offset portion spaced apart from the rigid PCB, and a transition portion connecting the contact portion to the offset portion. The dielectric region is positioned between the rigid PCB and the offset portion of the flexible PCB. The encapsulant adjoins at least a portion of the offset portion. The encapsulant is configured to allow a pressure or force applied thereto to transfer to and displace at least a portion of the offset portion towards the rigid PCB. The measurement device includes a converter and a controller. The converter is configured to convert a signal from the capacitive sensor to a digital signal. The controller is configured to process the digital signal and output pressure data indicating a pressure detected by the sensor assembly.

In various embodiments, the present disclosure provides a method for forming a catheter assembly. The method includes adhering an offset portion of a flexible PCB to a cap. The flexible PCB includes a contact portion and the offset portion. The cap includes an encapsulant adjoining at least a portion of the offset portion. The method also includes attaching the contact portion of the flexible PCB to a rigid printed circuit board (PCB) with the offset portion spaced apart from the rigid PCB. The rigid PCB is configured to retain a rigid shape. The method further includes positioning a dielectric region between the offset portion of the flexible PCB and the rigid PCB. The encapsulant is configured to allow a pressure or force applied thereto to transfer to and displace at least a portion of the offset portion towards the rigid PCB. The dielectric region is formed of a dielectric material configured to be compressed by the portion of the offset portion displaced towards the rigid PCB. The flexible PCB, the rigid PCB, the dielectric region, and the cap define a sensor assembly. The method also includes inserting the sensor assembly into an electronics lumen of a catheter. The catheter includes a catheter shaft and the electronics lumen formed in the catheter shaft.

### BRIEF DESCRIPTION OF THE DRAWINGS

For a detailed understanding of the present disclosure, reference should be made to the following detailed description, taken in conjunction with the accompanying drawings, in which like elements have generally been designated with like numerals, and wherein:
FIG. 1 is a block diagram of a capacitive catheter system, in accordance with one or more embodiments;
FIG. 2 is a cut-away perspective view of a portion of a catheter assembly of the capacitive catheter system of FIG. 1, in accordance with one or more embodiments;
FIG. 3 is a perspective view of a sensor assembly of the catheter assembly of FIG. 2, in accordance with one or more embodiments;
FIG. 4 is a cut-away perspective view of the sensor assembly of FIG. 3;
FIG. 5 is a side view of a capacitive sensor of the sensor assembly of FIGS. 3 and 4, in accordance with one or more embodiments;
FIG. 6 is a top view of a portion of a flexible PCB of the capacitor of FIG. 5, in accordance with one or more embodiments;
FIG. 7 is a block diagram of the sensor assembly of FIGS. 3 and 4 illustrating a layout of through-hole pads, in accordance with one or more embodiments;
FIG. 8 is a block diagram of the sensor assembly of FIGS. 3 and 4 illustrating a layout of through-hole pads, in accordance with one or more embodiments;
FIG. 9 is a sensor assembly of the catheter assembly of FIG. 2, in accordance with one or more embodiments; and
FIG. 10 is a flowchart of a method for forming a catheter assembly.

### DETAILED DESCRIPTION

In various embodiments, the present disclosure relates to pressure sensing catheter assemblies including a capacitive sensor (also referred to as capacitive catheter assemblies), catheter systems, and methods for forming catheter assemblies. The catheter assemblies include a sensor assembly. The sensor assembly includes a flexible PCB joined to a rigid printed circuit board (PCB) with a portion of the flexible PCB offset from the rigid PCB. A dielectric region is positioned between the offset portion and the rigid PCB, the offset portion, rigid PCB, and dielectric region defining a capacitive sensor, the offset portion configured to flex towards the rigid PCB and compress the dielectric region and upon application of an applied pressure. The offset portion is configured to restore to an initial position upon removal of the applied pressure. The sensor assembly may include an encapsulant positioned over at least a portion of the offset portion that is configured to transfer pressure applied to the catheter to the flexible PCB. A sensor assembly formed with a flexible PCB, a rigid PCB, a dielectric region, and an encapsulant may be simpler than off-the-shelf sensor solutions (e.g., Micro-Electro-Mechanical Systems (MEMS) pressure sensors, without limitation), which may simplify manufacturing and reduce manufacturing costs of a catheter system.

The illustrations presented herein are not actual views of any system, device, or structure, or any component thereof, but are merely idealized representations, which are employed to describe embodiments of the present invention.

As used herein, the singular forms following "a," "an," and "the" are intended to include the plural forms as well, unless the context clearly indicates otherwise.

As used herein, the term "may" with respect to a material, structure, feature, or method act indicates that such is contemplated for use in implementation of an embodiment of the disclosure, and such term is used in preference to the more restrictive term "is" so as to avoid any implication that other compatible materials, structures, features, and methods usable in combination therewith should or must be excluded.

As used herein, any relational term, such as "first," "second," "top," "bottom," "upper," "lower," "above," "beneath," "side," "upward," "downward," etc., is used for clarity and convenience in understanding the disclosure and accompanying drawings, and does not connote or depend on any specific preference or order, except where the context clearly indicates otherwise. For example, these terms may refer to an orientation of elements of any system, device, or structure, when utilized in a conventional manner. Furthermore, these terms may refer to an orientation of elements of any system, device, or structure, as illustrated in the drawings.

As used herein, the term "substantially" in reference to a given parameter, property, or condition means and includes to a degree that one skilled in the art would understand that the given parameter, property, or condition is met with a small degree of variance, such as within acceptable manufacturing tolerances. By way of example, depending on the particular parameter, property, or condition that is substantially met, the parameter, property, or condition may be at least 90.0% met, at least 95.0% met, at least 99.0% met, or even at least 99.9% met.

As used herein, the term "about" used in reference to a given parameter is inclusive of the stated value and has the meaning dictated by the context (e.g., it includes the degree of error associated with measurement of the given parameter, as well as variations resulting from manufacturing tolerances, etc.).

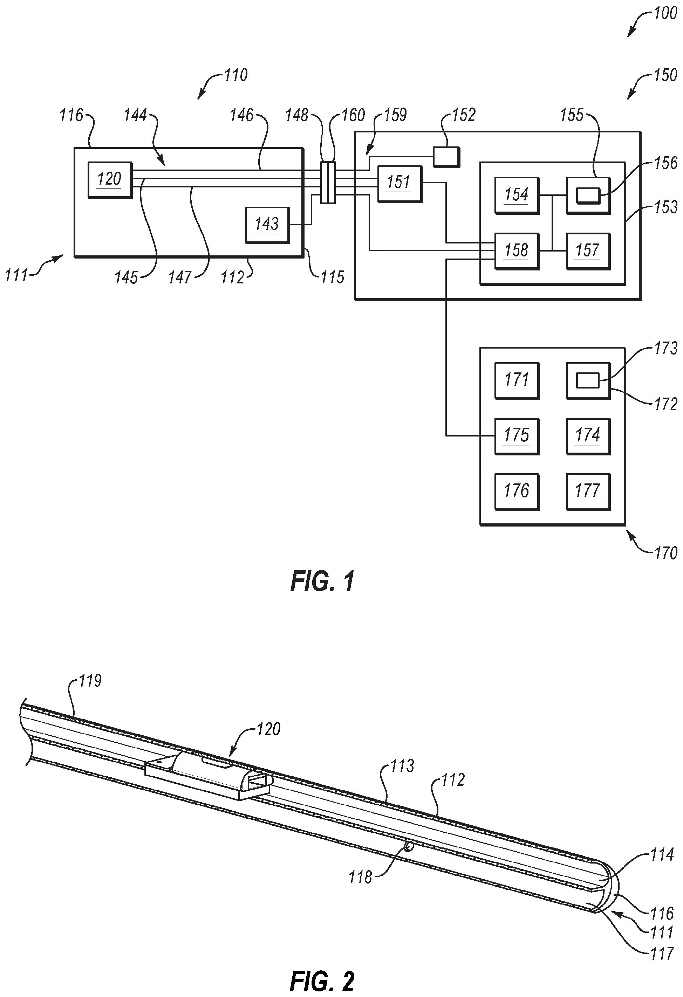

FIG. 1 is a block diagram of a capacitive catheter system 100, in accordance with one or more embodiments. FIG. 2 is a cut-away perspective view of a portion of a catheter assembly of the capacitive catheter system of FIG. 1, in accordance with one or more embodiments. The capacitive catheter system 100 may be utilized for manometry within various parts of the gastrointestinal tract (e.g., esophageal manometry, anorectal manometry, or urology manometry, without limitation). Referring to FIGS. 1 and 2, in various embodiments, the capacitive catheter system 100 includes a catheter assembly 110, a measurement device 150, and an access device 170.

The catheter assembly 110 includes a catheter 111, one or more sensor assemblies 120, electrical conductors 144, and a connector 148. The catheter 111 includes a catheter shaft 112 and an electronics lumen 114. The catheter shaft 112 includes a catheter wall 113 generally defining a tube shape extending from a proximal end 115 to a distal end 116 of the catheter 111. The catheter 111 may include an atraumatic tip at a distal end 116 of the catheter shaft 112, which is distal to a signal connection to the measurement device 150. The distal end 116 is configured to be inserted into a patient's body with minimal tissue damage and configured to avoid causing injury or trauma to the patient's body. The proximal end 115 is configured to remain outside of the patient's body.

The electronics lumen 114 is formed in the catheter shaft 112 and defines a cavity extending longitudinally within the catheter wall 113 extending from the proximal end 115 towards the distal end 116 and may extend to the distal end 116. The electronics lumen 114 is configured to receive the sensor assembly 120 therein and is configured for the electrical conductors 144 to pass therethrough from the sensor assembly 120, out of the proximal end 115, to the connector 148. The connector 148 is configured to form the signal connection with a connector 160 of the measurement device 150 for communication between the catheter assembly 110 and the measurement device 150. The signal connection may be any type of signal connection or connector known in the art. Any electromechanical coupler may be employed in accordance with embodiments of the present disclosure. While a single electronics lumen 114 is shown, the catheter 111 may include multiple electronics lumens 114, each configured to receive one or more sensor assemblies 120.

In various embodiments, the catheter 111 includes a fluids lumen 117 and one or more ports 118. The fluids lumen 117 is formed in the catheter shaft 112 and defines a cavity extending longitudinally within the catheter wall 113, adjacent to and separate from the electronics lumen, extending from the proximal end 115 towards the distal end 116 and may extend to the distal end 116. The fluids lumen 117 is configured to transport fluids therethrough (e.g., introduce fluids into the body, such as medicaments into the bladder, or allowing urine to drain out of the bladder, without limitation). The one or more ports 118 is formed in and extends through the catheter wall 113 from the fluids lumen 117 to an outer surface of the catheter wall 113. The one or more ports 118 is configured to transport fluids between the fluids lumen 117 and the patient's body, such as the bladder. The one or more ports 118 may be positioned closer to the distal end 116 than the proximal end 115, such as at or adjacent to the distal end 116. The one or more ports 118 may be positioned between the sensor assembly 120 and the distal end 116.

The sensor assembly 120 is positioned within the electronics lumen 114 closer to the distal end 116 than the proximal end 115. The positioning of the sensor assembly 120 or assemblies 120 relative to the distal end of the catheter may be dependent upon the type of procedure for which the capacitive catheter system 100 is to be employed. While a single sensor assembly 120 is shown in FIGS. 1 and 2, any number of sensor assemblies 120 may be positioned within the catheter 111, such as arranged along the axis of the catheter 111 or arranged circumferentially about the catheter 111 within either a single electronics lumen 114 or respectively positioned within separate electronics lumens 114.

The sensor assembly 120 is configured to measure pressure within a patient's body (e.g., urinary pressure, such as bladder pressure, respiratory pressure, such as airway pressure, intra-abdominal pressure, such as abdominal cavity pressure, intracranial pressure, and cardiovascular pressure, such as heart and blood vessel pressure, without limitation).

The electrical conductors 144 extend from the sensor assembly 120 through the electronics lumen 114 and out of the catheter 111 at or adjacent to the proximal end 115 and terminate at the connector 148. The electrical conductors 144 may include a signal conductor 145, a driven shield conductor 146, and a ground 147. The signal conductor 145 is configured to conduct a primary drive signal between the sensor assembly 120 and the measurement device 150. The driven shield conductor 146 is configured to isolate the sensor assembly 120 from outside interference and parasitic capacitances from forming in undesired locations. In various embodiments, the driven shield conductor 146 is positioned outside of the signal conductor 145 relative to ground 147. The electrical conductors 144 may be conductors within a long flexible PCB or shielded wires, without limitation. The signal connection formed by joining the connector 148 and the connector 160 connects the signal conductor 145 and the ground 147 to the converter 151 and connects the driven shield conductor 146 to a driven shield signal source 152 via electrical conductors 159 of the measurement device 150.

In various embodiments, the catheter assembly 110 includes data storage 143. The data storage 143 comprises non-volatile memory (e.g., an on-board memory module, such as EEPROM, without limitation). The data storage 143 is configured to store calibration data of each sensor assembly 120 of the catheter assembly 110. The calibration data may include a transfer function defining a relationship between an applied pressure and an output capacitance, which may be determined in a calibration process.

In various embodiments, the catheter assembly 110 includes depth indicators 119. The depth indicators 119 may include measurement marks along the catheter shaft 112 providing visual marks for indicating and allowing verification of a position of the catheter 111 within the patient's body.

The measurement device 150 includes a converter 151 and a controller 153. The converter 151 is configured to convert the signal from the sensor assembly 120 to a digital signal for processing by the controller 153. In various embodiments, the converter 151 includes a capacitance to digital converter.

In various embodiments, the controller 153 (e.g., a microcontroller, without limitation) includes memory 155, one or more processors 154, data storage 157, and communication equipment 158. In various embodiments, the memory 155 is configured for storing instructions 156 for execution by the one or more processors 154. The one or more processors 154 are configured to execute the instructions 156 stored by the memory 155. The instructions 156, when executed by the one or more processors 154, may enable the one or more processors 154 to process and interpret the signal received from the sensor assembly 120 (e.g., the digital signal converted from the capacitive signal by the converter 151) to output pressure data indicating a pressure detected by the sensor assembly 120. The data storage 157 includes non-volatile memory and is configured to receive and store the calibration data from the data storage 143. The calibration data may be utilized in the processing and interpretation of the signal received from the sensor assembly 120.

The communication equipment 158 is configured to facilitate communication (e.g., wired or wireless communication, without limitation) between the controller 153 and other devices (e.g., the access device 170 and the catheter assembly 110). The communication may be wired or wireless communication and may utilize any suitable communication protocol such as a wired protocol (e.g., an Ethernet protocol or a universal serial bus protocol (USB), without limitation) or a wireless networking protocol (e.g., Wi-Fi^{®}, without limitation), a short-range wireless protocol (e.g., BLUETOOTH^{®}, without limitation), a near-field communication standard, or any other wireless optical or radio-frequency protocol. In various embodiments, the communication equipment 158 includes one or more antennas for the wireless communication. In various embodiments, the communication equipment 158 includes an Internet of Things (IOT) Subscriber Identity Module (SIM) card (e.g., a machine-to-machine SIM card, or a Universal Integrated Circuit Card, without limitation).

The memory 155, one or more processors 154, communication equipment 158, and data storage 157 may be on and electrically connected via the PCB.

In various embodiments, the access device 170 includes memory 172, one or more processors 171, data storage 174, communication equipment 175, a display 176, and an input device 177. The memory 172, the one or more processors 171, the data storage 174, and the communication equipment 175 of the access device 170 may be the same or similar to the memory 155, the one or more processors 154, the data storage 157, and the communication equipment 158 of the controller 153. The memory 172 is configured for storing instructions 173. The instructions 173, when executed by the one or more processors 171, may enable the one or more processors 171 to obtain the pressure data from the controller 153 and cause the pressure data to be displayed on the display 176 (e.g., within a user interface, without limitation). The input device 177 (e.g., a keyboard or touchscreen, without limitation) is configured to receive inputs from the user, which may be used to control operation of the catheter assembly 110 and control the display of the pressure data.

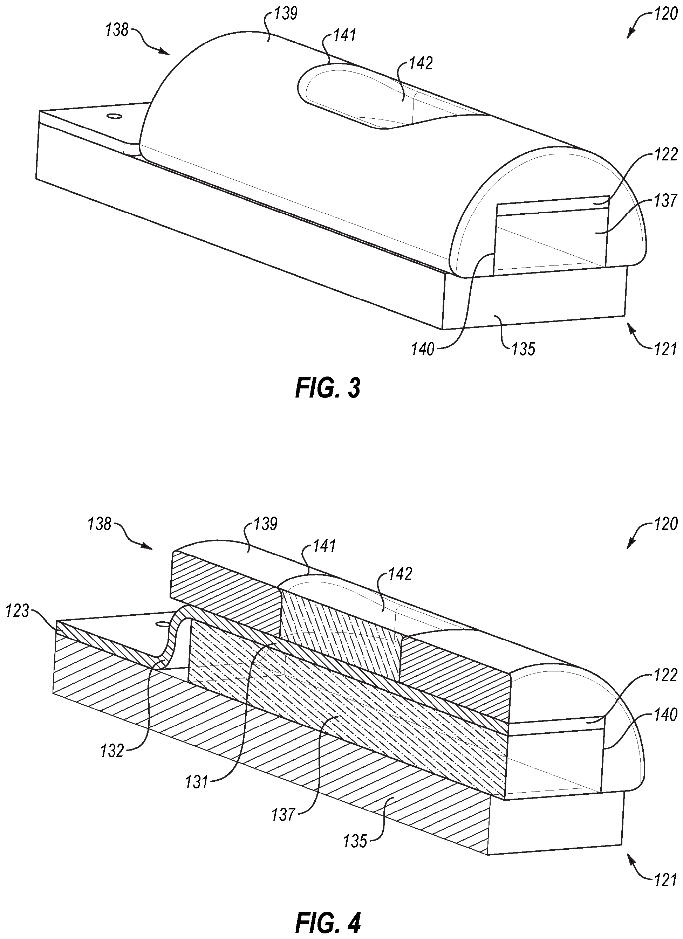

FIG. 3 is a perspective view of a sensor assembly 120 of the catheter assembly 110 of FIG. 2, in accordance with one or more embodiments. FIG. 4 is a cut-away perspective view of the sensor assembly 120 of FIG. 3. Referring to FIGS. 3 and 4, in various embodiments, the sensor assembly 120 includes a rigid PCB 135, a flexible PCB 122, and a dielectric region 137. The rigid PCB 135 includes a solid PCB structure that retains a rigid shape.

The flexible PCB 122 may be flexible in an elastically deformable sense and/or in a plastically deformable sense. The flexible PCB 122 includes a contact portion 123, an offset portion 131, and a transition portion 132. The contact portion 123 is joined to the rigid PCB 135, the offset portion 131 is offset and spaced apart from the rigid PCB 135, and the transition portion 132 connects the offset portion 131 to the contact portion 123. The offset portion 131 is arranged at an elevation different than that of the contact portion 123 in a direction transverse to the longitudinal direction. The transition portion 132 may include one or more bends or folds. In various embodiments, each of the contact portion 123 and the offset portion 131 extends substantially parallel to the rigid PCB 135. In various embodiments, a distance between the offset portion 131 and the rigid PCB 135 may be in a range from about 0.01 mm to about 0.20 mm, more particularly from about 0.05 mm to about 0.15 mm, such as about 0.1 millimeter for example. The flexible PCB 122 may generally include an S shape and the transition portion 132 may generally define the curves of the S shape.

The dielectric region 137 is positioned between the offset portion 131 and the rigid PCB 135 and may generally fill a gap between the rigid PCB 135 and the offset portion 131. The offset portion 131, the rigid PCB 135, and the dielectric region 137 generally define a capacitive sensor 121. The dielectric region 137 may include a compressible dielectric material (e.g., dielectric polymers (e.g., elastomers), such as silicone, polydimethylsiloxane (PDMS), etc., without limitation) that is configured with a hardness that allows the offset portion 131 to flex from and restore to an initial position upon application and removal of an applied pressure/force. In various embodiments, the material of the dielectric region 137 may exhibit hardness in a range extending from about 10 on the Shore 00 scale to about 20 on the Shore A scale.

In various embodiments, the sensor assembly 120 includes a cap 138. The cap 138 includes an encapsulant 142 positioned over at least a portion of the offset portion 131 of the flexible PCB 122 opposite the dielectric region 137. The encapsulant 142 is configured to allow a pressure or force applied thereto to transfer to and displace at least a portion of the offset portion 131 towards the rigid PCB 135. The encapsulant 142 may be configured to flex and/or compress towards the offset portion 131 while a pressure is applied and restore to a default shape when a pressure is not applied thereto.

In various embodiments, the cap 138 includes a cap body 139, a slot 140, and an opening 141. The cap body 139 includes a horizontal circular segment shape with a portion of a cylindrical surface and a base. The slot 140 is formed in the cap body 139 extending across the base parallel to an axis of the horizontal circular segment shape. The slot 140 receives the offset portion 131 of the flexible PCB 122 and the dielectric region 137 therein. The portion of the cylindrical surface has a radius substantially similar to a radius of an inner surface of the catheter shaft 112 defined by the electronics lumen 114. The flexible PCB 122 (e.g., an outer/top surface of the offset portion 131, without limitation) is adhered to the cap body 139 via an adhesive (e.g., a polymeric adhesive such as an epoxy, silicon, a thermoplastic material, etc.). In various embodiments, the cap body 139 includes a rigid material (e.g., a metal or metal alloy such as stainless steel, a rigid thermoplastic material, *etc.*)*.*

The opening 141 formed in the cap body 139 extending through the horizontal circular segment shape that is positioned over the offset portion 131 of the flexible PCB 122. The opening 141 may be centered over a center of a length of the flexible PCB 122. In these various embodiments, the encapsulant 142 is positioned within the opening 141. In various embodiments, each end of the offset portion 131 of the flexible PCB 122 is joined to the cap body 139 and a portion of the flexible PCB 122 overlapping the opening 141 is free from the cap body 139, which allows the portion of the flexible PCB 122 to displace relative to the cap body 139 upon application of a pressure or force thereto. In various embodiments, the offset portion 131 is adhered to the cap body 139 adjacent to each longitudinal end of the opening 141. These attachment points may keep the offset portion 131 of the flexible PCB 122 fixed to the cap body 139 while the opening in the center allows the offset portion 131 to deform with pressure.

With a portion of the flexible PCB 122 allowed to flex towards the rigid PCB 135, as pressure is applied to the flexible PCB 122 (e.g., pressure applied to the catheter 111, which is transferred to the flexible PCB 122 via the encapsulant 142, without limitation), the flexible PCB 122 will deform toward the rigid PCB 135, changing a capacitance of the capacitive sensor 121. The capacitive sensor 121 may follow the properties of a parallel plate capacitor where capacitance is defined as: $C = \frac{{∈}_{0} A}{d}$, where C is the capacitance, ε₀ is the electrostatic constant, A is the area, and d is a distance between the plates. The value of the capacitance relative to the pressure applied may be defined by a transfer function. The transfer function may be unique to each sensor assembly 120 and may be determined via a calibration process.

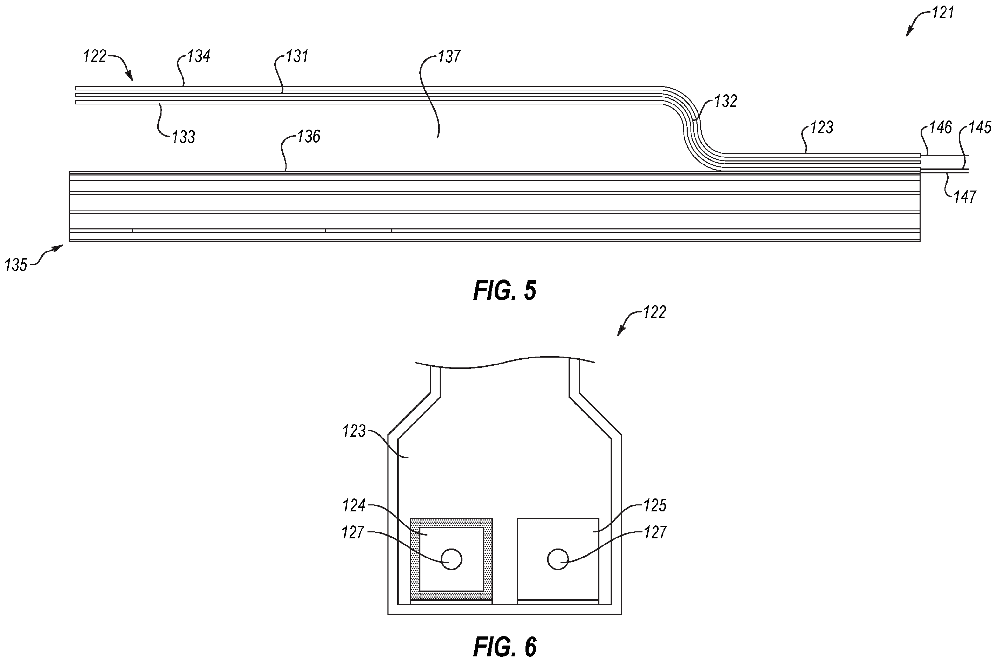

FIG. 5 is a side view of the capacitive sensor 121 of the sensor assembly 120 of FIGS. 3 and 4, in accordance with one or more embodiments. Referring to FIG. 5, in various embodiments, the flexible PCB 122 includes a multi-layer flexible PCB (e.g., a dual layer flexible PCB, without limitation). In various embodiments, the multi-layer flexible PCB includes a first layer 133 electrically connected to the signal conductor 145 and a second layer 134 electrically connected to the driven shield conductor 146. The second layer 134 is positioned outside of the first layer 133 (i.e., further from the dielectric region 137 and the rigid PCB 135 than the first layer 133). In some of these various embodiments, the first layer 133 is a bottom/innermost layer of the flexible PCB 122 adjacent to the dielectric region 137 in the offset portion 131 and closest to the rigid PCB 135. A non-conductive dielectric layer may separate the first layer 133 and the second layer 134. The second layer 134 is configured to generate a driven shield to isolate the sensor assembly 120, and in particular, capacitive sensor 121, from outside interference and parasitic capacitances.

In various embodiments, the rigid PCB 135 includes a multi-layer rigid PCB (e.g., a dual layer, triple layer, or quadruple layer rigid PCB, without limitation). In various embodiments, the multi-layer rigid PCB includes a ground layer 136 electrically connected to the ground 147. In some of these various embodiments, the ground layer 136 is a top/innermost layer of the rigid PCB 135 adjacent to the dielectric region 137 and closest to the flexible PCB 122. In alternative embodiments, the connections could be inverted with the rigid PCB 135 connected to signal and the flexible PCB 122 connected to ground.

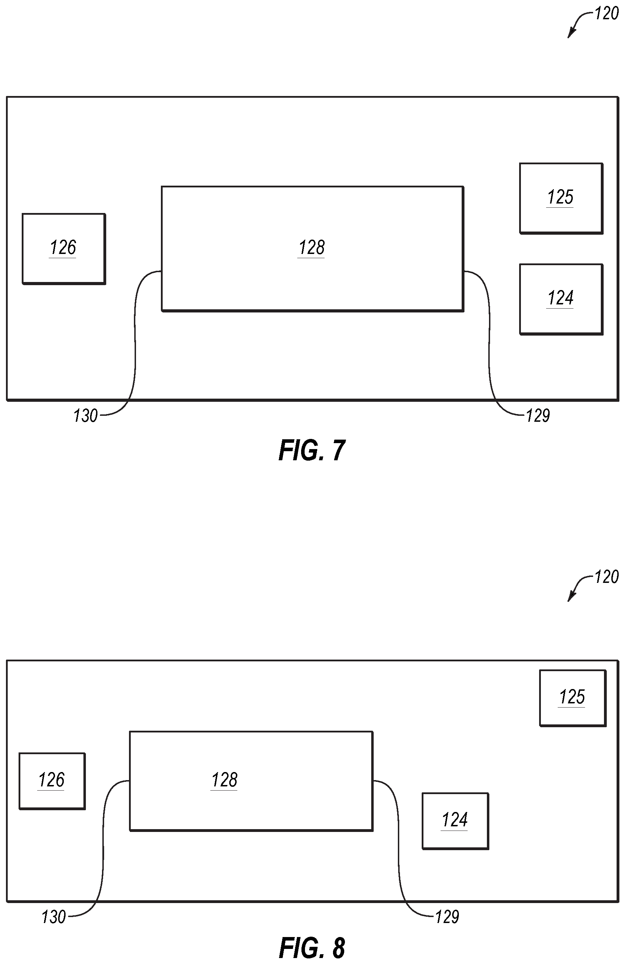

FIG. 6 is a top view of a portion of the flexible PCB 122 of the capacitor of FIG. 5, in accordance with one or more embodiments. FIG. 7 is a block diagram of the sensor assembly of FIGS. 3 and 4 illustrating a layout of through-hole pads (also referred to as bond pads), in accordance with one or more embodiments. FIG. 8 is a block diagram of the sensor assembly of FIGS. 3 and 4 illustrating a layout of through-hole pads, in accordance with one or more embodiments. Referring to FIGS. 6-8, in various embodiments, the contact portion 123 is joined to the rigid PCB 135 via through-hole pads 124, 125, 126. Each of the through-hole pads 124, 125, 126 includes a through-hole 127. The through-hole pads 124, 125, 126 may include a shield pad 124 for a connection of the driven shield conductor 146 to the flexible PCB 122, a signal pad 125 for a connection of the signal conductor 145 to the flexible PCB 122, and a ground pad 126 for a connection of the rigid PCB 135 to ground 147. The contact portion 123 may also be joined to the rigid PCB 135 via an adhesive (e.g., solder paste, without limitation). The through-hole pads may be sized large enough to eliminate the need for the use of wire-bonding tools for forming a connection therewith, which may allow for soldering the connection by hand or for an automated soldering process.

In various embodiments, the shield pad 124 and the signal pad 125 are positioned adjacent to a first longitudinal end 129 of a sensing region 128, and the ground pad 126 is positioned adjacent to a second longitudinal end 130 of the sensing region 128, which is an end opposite the first longitudinal end 129 in the longitudinal direction. The sensing region 128 may be defined by an area of the capacitive sensor 121 that is capable of sensing pressure (e.g., the portion of the flexible PCB 122 displace relative to the cap body 139, without limitation).

Referring to FIG. 7, in various embodiments, the shield pad 124 and signal pad 125 are adjacent to one another and positioned about equidistant from the first longitudinal end 129 of the sensing region 128. Referring to FIG. 8, in other various embodiments, one of the shield pad 124 and the signal pad 125 is positioned further from the first longitudinal end 129 than an other of the shield pad 124 and the signal pad 125 in at least the longitudinal direction. One or more of the shield pad 124 and the signal pad 125 may be offset laterally and positioned adjacent to a side of the sensor assembly 120 to increase a distance between the shield pad 124 and the signal pad 125. The layout of the through-hole pads (e.g., the layouts of FIG. 7 and FIG. 8, without limitation) may be configured to maximize a distance between the through-hole pads, which may limit the risk of solder bridges, resulting in easier connections to form during the manufacturing process.

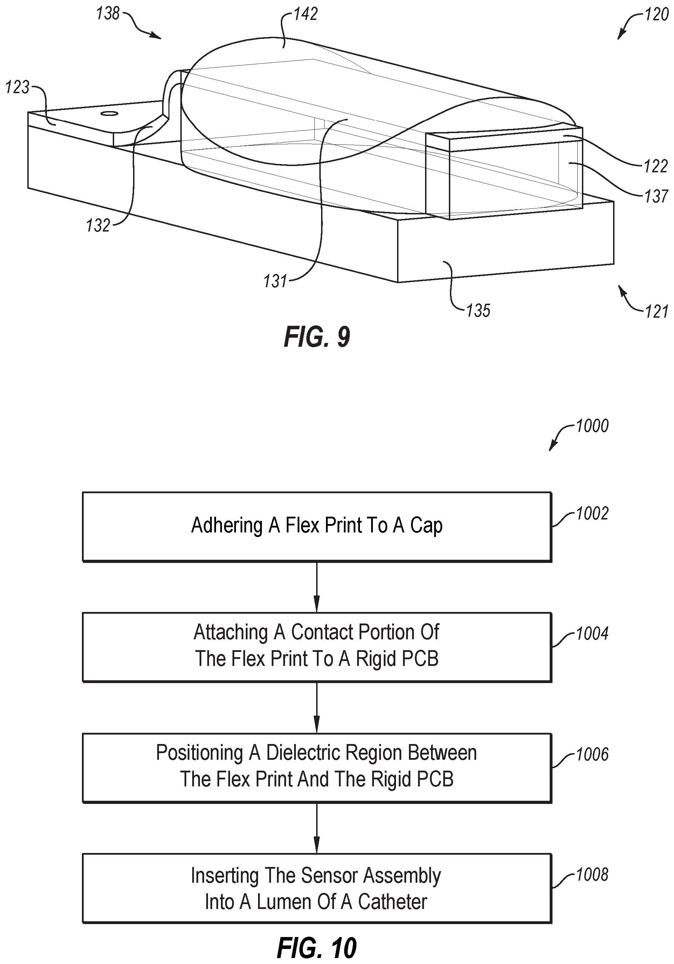

FIG. 9 is a sensor assembly 120 of the catheter assembly 110 of FIG. 2, in accordance with one or more embodiments. As noted above, the cap 138 includes an encapsulant 142 positioned over at least a portion of the offset portion 131 of the flexible PCB 122. Referring to FIG. 9, in various embodiments, the encapsulant 142 extends over the offset portion 131 at least for a length of the dielectric region 137 (e.g., from adjacent to or at a distal end of the offset portion 131 to adjacent to or at a proximal end of the offset portion 131 that is proximal to the transition portion 132, without limitation). The encapsulant 142 may extend down each side of the dielectric region 137 to a top/inner surface of the rigid PCB 135 (e.g., a surface of the ground layer 136, without limitation). An outer surface of the encapsulant 142 may include a cylindrical shape (e.g., a right circular cylinder) that includes a radius substantially similar to a radius of an inner surface of the catheter shaft 112 defined by the electronics lumen 114.

FIG. 10 is a flowchart of a method 1000 for forming a catheter assembly. The catheter assembly may be any embodiment of the catheter assembly 110 disclosed herein. The method 1000 includes adhering a flexible PCB 122 to a cap 138 at act 1002. The flexible PCB may be adhered to the cap via an adhesive. The flexible PCB includes one or more layers and may include at least two layers (e.g., the first layer 133 and the second layer 134, without limitation). Act 1002 may include adhering an outer layer (e.g., the second layer 134, without limitation) to the cap.

In various embodiments, the cap includes a cap body (e.g., cap body 139, without limitation) with an opening (e.g., opening 141) formed therein and act 1002 may include adhering an offset portion (e.g., offset portion 131, without limitation) of the flexible PCB to the cap body adjacent to each longitudinal end of the opening. The offset portion of the flexible PCB may be adhered to the cap at other locations offset from the opening. Other portions of the flexible PCB may also be adhered to the cap.

In various embodiments, the cap is primarily or completely formed of an encapsulant. In these various embodiments, the flexible PCB is adhered to the encapsulant at one or more points along a longitudinal length of the offset portion of the flexible PCB.

The method 1000 includes attaching a contact portion of the flexible PCB to a rigid PCB (e.g., the rigid PCB 135, without limitation) at act 1004. The contact portion (e.g., the contact portion 123, without limitation), arranged at an elevation different than that of the offset portion, in a direction transverse to the longitudinal direction, is joined to the rigid PCB with the offset portion spaced apart from the rigid PCB. In various embodiments, the contact portion is soldered to the rigid PCB via solder pads.

In various embodiments, the method 1000 includes adhering the cap (e.g., a base of the cap body or the encapsulant) to the rigid PCB (e.g., to lateral sides of the rigid PCB or to edges of a top surface of the rigid PCB, such as a surface of the ground layer 136, without limitation).

The method 1000 includes positioning a dielectric region (e.g., dielectric region 137, without limitation) between the flexible PCB and the rigid PCB at act 1006. In various embodiments, the dielectric region is affixed to one of the flexible PCB or the rigid PCB prior to act 1004 and may be affixed to the other of the flexible PCB or the rigid PCB during act 1004.

Forming the catheter assembly includes forming a sensor assembly, which includes acts 1002, 1004, and 1006.

The method 1000 includes inserting the sensor assembly into a lumen (e.g., the electronics lumen 114, without limitation) of a catheter (e.g., catheter 111) at act 1008. The sensor assembly may be secured to the catheter assembly within the lumen (e.g., via an adhesive, such as glue, or via an interference fit, such as an outer diameter of the cap being larger than an inner diameter defined by the lumen, without limitation).

In various embodiments, the method 1000 includes forming an electrical connection between the flexible PCB and a first conductor (e.g., signal conductor 145, without limitation) configured to connect the flexible PCB to a primary drive signal, forming an electrical connection between the flexible PCB and a second conductor (e.g., a driven shield conductor 146, without limitation) configured to connect the flexible PCB to a driven shield signal, and forming an electrical connection between the rigid PCB and a third conductor (e.g., a ground, without limitation). The first conductor may be connected to an inner layer (e.g., the first layer 133, without limitation) of the flexible PCB, the second conductor may be connected to an outer layer (e.g., the second layer 134, without limitation) of the flexible PCB, and the third conductor may be connected to an inner layer (e.g., the ground layer 136, without limitation) of the rigid PCB.

The method 1000 may include calibrating the sensor assembly. Calibrating the sensor assembly may include obtaining calibration data by applying known pressures to the catheter assembly at a location of the sensor assembly and capturing capacitance values while the known pressures are applied. Calibrating the sensor assembly may also include defining a transfer function based on the capacitance values captured. The method 1000 may include storing the calibration data (e.g., the transfer function, without limitation)

Calibration data may include a transfer function defining a relationship between an applied pressure and an output capacitance in a data storage (e.g., non-volatile memory positioned within the catheter body, without limitation) of the catheter assembly.

A sensor assembly as disclosed herein inserted into a catheter lumen may simplify manufacturing and may reduce manufacturing costs of a catheter system.

Non-limiting example embodiments of the present disclosure may include:
Embodiment 1: A catheter assembly comprising: a catheter comprising: a catheter shaft; and an electronics lumen formed in the catheter shaft; and a sensor assembly inserted into the electronics lumen, the sensor assembly comprising: a rigid printed circuit board (PCB) that retains a rigid shape; a flexible PCB comprising a contact portion joined to the rigid PCB and an offset portion spaced apart from the rigid PCB; a dielectric region positioned between the rigid PCB and the offset portion of the flexible PCB; and a cap comprising an encapsulant adjoining at least a portion of the offset portion, the encapsulant configured to allow a pressure or force applied thereto to transfer to and displace at least a portion of the offset portion towards the rigid PCB.
Embodiment 2: The catheter assembly according to Embodiment 1, wherein: the flexible PCB comprises a first layer and a second layer, the first layer positioned closest to the rigid PCB and the second layer positioned further from the rigid PCB than the first layer; and the rigid PCB comprises multiple layers including a ground layer positioned closest to the flexible PCB.
Embodiment 3: The catheter assembly according to any of Embodiments 1 and 2, further comprising: a signal conductor connected to the first layer configured to conduct a primary drive signal between the sensor assembly and a measurement device; a driven shield conductor connected to the second layer configured to isolate the sensor assembly from outside interference; and a ground conductor configured to connect the ground layer to ground.
Embodiment 4: The catheter assembly according to any of Embodiments 1 to 3, wherein the encapsulant extends over the offset portion at least for a length of the dielectric region and extends down each side of the dielectric region to a top surface of the rigid PCB.
Embodiment 5: The catheter assembly according to any of Embodiments 1 to 4, wherein an outer surface of the encapsulant includes a cylindrical shape that includes a radius substantially similar to a radius of an inner surface of the catheter shaft defined by the electronics lumen.
Embodiment 6: The catheter assembly according to any of Embodiments 1 to 5, wherein the cap further comprises: a cap body including a horizontal circular segment shape including a portion of a cylindrical surface and a base, the portion of the cylindrical surface having a radius substantially similar to a radius of an inner surface of the catheter shaft defined by the electronics lumen; a slot formed in the cap body extending across the base parallel to an axis of the horizontal circular segment shape, the slot receiving the offset portion and the dielectric region therein; and an opening formed in the cap body extending through the horizontal circular segment shape, the opening positioned over the offset portion of the flexible PCB, wherein the encapsulant is positioned within the opening.
Embodiment 7: The catheter assembly according to any of Embodiments 1 to 6, wherein the offset portion is adhered to the cap body adjacent to each longitudinal end of the opening.
Embodiment 8: A catheter system comprising: a catheter comprising: a catheter shaft; and an electronics lumen formed in the catheter shaft; and a sensor assembly inserted into the electronics lumen, the sensor assembly comprising: a capacitive sensor comprising: a rigid printed circuit board (PCB) that retains a rigid shape; a flexible PCB comprising a contact portion joined to the rigid PCB, an offset portion spaced apart from the rigid PCB, and a transition portion connecting the contact portion to the offset portion; and a dielectric region positioned between the rigid PCB and the offset portion of the flexible PCB; and an encapsulant adjoining at least a portion of the offset portion, the encapsulant configured to allow a pressure or force applied thereto to transfer to and displace at least a portion of the offset portion towards the rigid PCB; and a measurement device comprising: a converter configured to convert a signal from the capacitive sensor to a digital signal; and a controller configured to process the digital signal and output pressure data indicating a pressure detected by the sensor assembly.
Embodiment 9: The catheter system according to Embodiment 8, wherein: the flexible PCB comprises a first layer and a second layer, the first layer positioned closest to the rigid PCB and the second layer positioned further from the rigid PCB than the first layer; and the rigid PCB comprises multiple layers including a ground layer positioned closest to the flexible PCB.
Embodiment 10: The catheter system according to any of Embodiments 8 and 9, wherein: the sensor assembly further comprises: a signal conductor connected to the first layer configured to conduct a primary drive signal between the sensor assembly and the measurement device; a driven shield conductor connected to the second layer configured to isolate the sensor assembly from outside interference; and a ground conductor; and the catheter system further comprising a connector joining the signal conductor and the ground conductor to the converter and joining the driven shield conductor to a signal source.
Embodiment 11: The catheter system according to any of Embodiments 8 to 10, wherein the encapsulant extends over the offset portion at least for a length of the dielectric region and extends down each side of the dielectric region to a top surface of the rigid PCB.
Embodiment 12: The catheter system according to any of Embodiments 8 to 11, wherein an outer surface of the encapsulant includes a cylindrical shape that includes a radius substantially similar to a radius of an inner surface of the catheter shaft defined by the electronics lumen.
Embodiment 13: The catheter system according to any of Embodiments 8 to 12, wherein the sensor assembly comprises a cap, the cap comprising: a cap body including a horizontal circular segment shape including a portion of a cylindrical surface and a base, the portion of the cylindrical surface having a radius substantially similar to a radius of an inner surface of the catheter shaft defined by the electronics lumen; a slot formed in the cap body extending across the base parallel to an axis of the horizontal circular segment shape, the slot receiving the offset portion and the dielectric region therein; and an opening formed in the cap body extending through the horizontal circular segment shape, the opening positioned over the offset portion of the flexible PCB, wherein the encapsulant is positioned within the opening.
Embodiment 14: The catheter system according to any of Embodiments 8 to 13, wherein each end of the offset portion of the flexible PCB is joined to the cap body and a portion of the flexible PCB overlapping the opening is free from the cap body.
Embodiment 15: A method for forming a catheter assembly, the method comprising: adhering an offset portion of a flexible PCB to a cap, the flexible PCB comprising a contact portion and the offset portion, and the cap comprising an encapsulant adjoining at least a portion of the offset portion; attaching the contact portion of the flexible PCB to a rigid printed circuit board (PCB) with the offset portion spaced apart from the rigid PCB, the rigid PCB configured to retain a rigid shape; positioning a dielectric region between the offset portion of the flexible PCB and the rigid PCB, wherein the encapsulant configured to allow a pressure or force applied thereto to transfer to and displace at least a portion of the offset portion towards the rigid PCB and the dielectric region is formed of a dielectric material configured to be compressed by the portion of the offset portion displaced towards the rigid PCB, and wherein the flexible PCB, the rigid PCB, the dielectric region, and the cap define a sensor assembly; and inserting the sensor assembly into an electronics lumen of a catheter, the catheter comprising a catheter shaft and the electronics lumen formed in the catheter shaft.
Embodiment 16: The method according to Embodiment 15, wherein the cap further comprises: a cap body including a horizontal circular segment shape including a portion of a cylindrical surface and a base, the portion of the cylindrical surface having a radius substantially similar to a radius of an inner surface of the catheter shaft defined by the electronics lumen; a slot formed in the cap body extending across the base parallel to an axis of the horizontal circular segment shape, the slot receiving the offset portion and the dielectric region therein; and an opening formed in the cap body extending through the horizontal circular segment shape, the opening positioned over the offset portion of the flexible PCB, wherein the encapsulant is positioned within the opening, and wherein adhering the offset portion of the flexible PCB to the cap includes adhering the offset portion to the cap body adjacent to each longitudinal end of the opening.
Embodiment 17: The method according to any of Embodiments 15 and 16, further comprising adhering the base of the cap body to the rigid PCB.
Embodiment 18: The method according to any of Embodiments 15 to 17, wherein the flexible PCB comprises a first layer and a second layer, the first layer positioned closest to the rigid PCB and the second layer positioned further from the rigid PCB than the first layer, and wherein the rigid PCB comprises multiple layers including a ground layer positioned closest to the flexible PCB, the method further comprising: forming an electrical connection between the first layer and a first conductor configured to connect the flexible PCB to a primary drive signal; forming an electrical connection between the second layer and a second conductor configured to connect the flexible PCB to a driven shield signal; and forming an electrical connection between the ground layer and a third conductor.
Embodiment 19: The method according to any of Embodiments 15 to 19, further comprising: forming an electrical connection between the flexible PCB and a first conductor configured to connect the flexible PCB to a primary drive signal; forming an electrical connection between the flexible PCB and a second conductor configured to connect the flexible PCB to a driven shield signal; and forming an electrical connection between the rigid PCB and a third conductor configured to connect the rigid PCB to ground.
Embodiment 20: The method according to Embodiments 19, wherein: the flexible PCB comprises a first layer and a second layer, the first layer positioned closest to the rigid PCB and the second layer positioned further from the rigid PCB than the first layer; the rigid PCB comprises multiple layers including a ground layer positioned closest to the flexible PCB; and wherein the first conductor is electrically connected to the first layer, the second conductor is electrically connected to the second layer, and the third conductor is electrically connected to the ground layer.

The embodiments of the disclosure described above and illustrated in the accompanying drawings do not limit the scope of the disclosure, which is encompassed by the scope of the appended claims and their legal equivalents. Any equivalent embodiments are within the scope of this disclosure. Indeed, various modifications of the disclosure, in addition to those shown and described herein, such as alternate useful combinations of the elements described, will become apparent to those skilled in the art from the description. Such modifications and embodiments also fall within the scope of the appended claims and equivalents.

## Claims

1. A catheter assembly comprising:
a catheter comprising:
a catheter shaft; and
an electronics lumen formed in the catheter shaft; and
a sensor assembly inserted into the electronics lumen, the sensor assembly comprising:
a rigid printed circuit board, PCB, that retains a rigid shape;
a flexible PCB comprising a contact portion joined to the rigid PCB and an offset portion spaced apart from the rigid PCB;
a dielectric region positioned between the rigid PCB and the offset portion of the flexible PCB; and
an encapsulant adjoining at least a portion of the offset portion, the encapsulant configured to allow a pressure or force applied thereto to transfer to and displace at least a portion of the offset portion towards the rigid PCB.

2. The catheter assembly of claim 1, wherein:
the flexible PCB comprises a first layer and a second layer, the first layer positioned closest to the rigid PCB and the second layer positioned further from the rigid PCB than the first layer; and
the rigid PCB comprises multiple layers including a ground layer positioned closest to the flexible PCB.

3. The catheter assembly of claim 2, further comprising:
a signal conductor connected to the first layer configured to conduct a primary drive signal between the sensor assembly and a measurement device;
a driven shield conductor connected to the second layer configured to isolate the sensor assembly from outside interference; and
a ground conductor configured to connect the ground layer to ground.

4. The catheter assembly of any one of the preceding claims, wherein the encapsulant extends over the offset portion at least for a length of the dielectric region and extends down each side of the dielectric region to a top surface of the rigid PCB.

5. The catheter assembly of claim 4, wherein an outer surface of the encapsulant includes a cylindrical shape that includes a radius substantially similar to a radius of an inner surface of the catheter shaft defined by the electronics lumen.

6. The catheter assembly of any one of the preceding claims, wherein the encapsulant forms a cap, wherein the cap preferably further comprises:
a cap body including a horizontal circular segment shape including a portion of a cylindrical surface and a base, the portion of the cylindrical surface having a radius substantially similar to a radius of an inner surface of the catheter shaft defined by the electronics lumen;
a slot formed in the cap body extending across the base parallel to an axis of the horizontal circular segment shape, the slot receiving the offset portion and the dielectric region therein; and
an opening formed in the cap body extending through the horizontal circular segment shape, the opening positioned over the offset portion of the flexible PCB, wherein the encapsulant is positioned within the opening.

7. The catheter assembly of claim 6, wherein the offset portion is adhered to the cap body adjacent to each longitudinal end of the opening.

8. The catheter assembly of claim 6 or claim 7, wherein each end of the offset portion of the flexible PCB is joined to the cap body and a portion of the flexible PCB overlapping the opening is free from the cap body.

9. The catheter assembly of any one of the preceding claims, wherein the rigid PCB, the flexible PCB and the dielectric region form
a capacitive sensor, and wherein the flexible PCB further comprises a transition portion connecting the contact portion to the offset portion.

10. A catheter system comprising:
the catheter assembly of any one of the preceding claims; and
a measurement device comprising:
a converter configured to convert a signal from the capacitive sensor to a digital signal; and
a controller configured to process the digital signal and output pressure data indicating a pressure detected by the sensor assembly.

11. The catheter system of claim 10, wherein:
the sensor assembly further comprises:
a signal conductor connected to the first layer configured to conduct a primary drive signal between the sensor assembly and the measurement device;
a driven shield conductor connected to the second layer configured to isolate the sensor assembly from outside interference; and
a ground conductor; and
the catheter system further comprising a connector joining the signal conductor and the ground conductor to the converter and joining the driven shield conductor to a signal source.

12. A method for forming a catheter assembly, the method comprising:
adhering an offset portion of a flexible PCB to a cap, the flexible PCB comprising a contact portion and the offset portion, and the cap comprising an encapsulant adjoining at least a portion of the offset portion;
attaching the contact portion of the flexible PCB to a rigid printed circuit board (PCB) with the offset portion spaced apart from the rigid PCB, the rigid PCB configured to retain a rigid shape;
positioning a dielectric region between the offset portion of the flexible PCB and the rigid PCB, wherein the encapsulant is configured to allow a pressure or force applied thereto to transfer to and displace at least a portion of the offset portion towards the rigid PCB and the dielectric region is formed of a dielectric material configured to be compressed by the portion of the offset portion displaced towards the rigid PCB, and wherein the flexible PCB, the rigid PCB, the dielectric region, and the cap define a sensor assembly; and
inserting the sensor assembly into an electronics lumen of a catheter, the catheter comprising a catheter shaft and the electronics lumen formed in the catheter shaft.

13. The method of claim 12, wherein the cap further comprises:
a cap body including a horizontal circular segment shape including a portion of a cylindrical surface and a base, the portion of the cylindrical surface having a radius substantially similar to a radius of an inner surface of the catheter shaft defined by the electronics lumen;
a slot formed in the cap body extending across the base parallel to an axis of the horizontal circular segment shape, the slot receiving the offset portion and the dielectric region therein; and
an opening formed in the cap body extending through the horizontal circular segment shape, the opening positioned over the offset portion of the flexible PCB, wherein the encapsulant is positioned within the opening, and
wherein adhering the offset portion of the flexible PCB to the cap includes adhering the offset portion to the cap body adjacent to each longitudinal end of the opening.

14. The method of claim 13, further comprising adhering the base of the cap body to the rigid PCB.

15. The method of any one of claims 12 to 14, wherein the flexible PCB comprises a first layer and a second layer, the first layer positioned closest to the rigid PCB and the second layer positioned further from the rigid PCB than the first layer, and wherein the rigid PCB comprises multiple layers including a ground layer positioned closest to the flexible PCB,
the method further comprising:
forming an electrical connection between the first layer and a first conductor configured to connect the flexible PCB to a primary drive signal;
forming an electrical connection between the second layer and a second conductor configured to connect the flexible PCB to a driven shield signal; and
forming an electrical connection between the ground layer and a third conductor.

16. The method of any one of claims 12 to 15, further comprising:
forming an electrical connection between the flexible PCB and a first conductor configured to connect the flexible PCB to a primary drive signal;
forming an electrical connection between the flexible PCB and a second conductor configured to connect the flexible PCB to a driven shield signal; and
forming an electrical connection between the rigid PCB and a third conductor configured to connect the rigid PCB to ground.

17. The method of claim 16, wherein:
the flexible PCB comprises a first layer and a second layer, the first layer positioned closest to the rigid PCB and the second layer positioned further from the rigid PCB than the first layer;
the rigid PCB comprises multiple layers including a ground layer positioned closest to the flexible PCB; and
wherein the first conductor is electrically connected to the first layer, the second conductor is electrically connected to the second layer, and the third conductor is electrically connected to the ground layer.
